# EUROPEAN PATENT SPECIFICATION

(11) **EP 2 319 436 B1**
(45) Date of publication and mention of the grant of the patent: **13.02.2013**
(21) Application number: 09425446.3
(22) Date of filing: 06.11.2009
(51) Int. Cl.: A61B 17/64, F16B 2/06, F16B 7/04

(54) **Clamp for external orthopaedic fixing device**
Klemme für Fixateur externe
Pince pour fixateur externe

(43) Date of publication of application: 11.05.2011
(73) Proprietor: ORTHOFIX S.r.l., 37012 Bussolengo (Verona) (IT)
(72) Inventor: Venturini, Daniele, 37064 Povegliano Veronese (Verona) (IT); Bagnasco, Mara, 20134 Milano (IT)
(74) Representative: Botti, Mario

(56) References cited:
- WO-A1-03/068082
- US-A1- 2002 177 754
- US-A1- 2006 177 263
- US-A1- 2008 065 068
- US-A1- 2009 088 751

## Description

### Field of application

The present invention relates to a clamp for orthopaedic applications, more particularly intended for binding, especially temporarily, the component elements of an external fixing system.

More particularly the invention relates to a clamp for an orthopaedic external fixator, comprising:
- a first attachment, provided with two arms respectively external and internal in relation to the overall structure of the clamp, defining one with the other a lateral housing with C-shaped section suitable for housing a first connection bar of the orthopaedic external fixator;
- a second attachment designed to allow the connection of the clamp to a second component of the orthopaedic external fixator, other than the first connection bar;
- a tightening element, at least partially threaded, which traverses and connects the first and the second attachment along an axis of rotation transverse to the extension of the external and internal arms; the tightening of said tightening element bringing the clamp from a loosened configuration, at which the two attachments are reciprocally rotatable around the axis of rotation, to a tightened configuration, at which the relative orientation of the attachments is blocked.

### Prior art

In orthopaedics, techniques of external fixation for the osteosynthesis of bone segments are regularly used. These techniques provide for the surgical application of a rigid exoskeleton, in its simplest form made up of a set of bars articulated one with the other by means of special clamps, associated by means of endosseus screws or wires to the bone structure of the patient. Overall the mechanical system adopted takes the name of external fixator.

The diversity of therapeutic needs in today's medical environment have contributed to the differentiation of various types of fixators, each of which has technical and morphological features suitable for the use for which it is intended. Thus for certain applications, mostly traumatological and of an urgent nature, so-called rapid fixators have been developed, such fixators being characterised by simplicity and quick tightening.

Rapid fixators comprise structural bars connected to clamps of various types. Bar-bar clamps are known for example which are intended to connect the bars one to the other in the points of articulation of the structure; bar-screw clamps are designed to associate the bars with non-piercing screws anchored to the bone of the patient. Bar-wire clamps, designed to restrain bars and wires, are also known.

All these clamps are elements with a particularly critical design, having to reconcile the opposing needs of ease-of-use and stability of the tightening.

Some of the clamps of the type described and present in the prior art comprise two attachments or modules, both provided with a pair of arms defining a C-shaped housing and elastically deformable at their point of joining. Thanks to the elastic joint a connection bar (or else a bone screw, or a wire) of the fixator can be snap-inserted between the arms of each attachment.

The two attachments are also traversed by holes for a tightening screw, such holes traversing the arms orthogonally in an intermediate position between the joint and the housing of the bar or screw. In the assembled configuration of the clamp said attachments are set one on top of the other and made integral by means of the tightening screw which is inserted in said joining holes, engaging by screwing at a final threaded portion of the hole of the second attachment. Before tightening of the screw the two attachments are one on top of the other in a loosened configuration and free to rotate around an axis of rotation giving the clamp a double C configuration with relative orientation which can be set on planes angularly displaced one in relation to the other.

Consequently the provided tightening of the screw allows both fixing of the relative position of the two C-shaped modules and tightening of these modules around the bar (or bone screw or wire, according to the alternatives mentioned above), appropriately preventing axial sliding and rotation.

Clamps of the type described above are disclosed for instance in patent publications US 2008/0065068 and US 2009/0088751.

The clamp of the type described above, although satisfactorily performing the function for which it is designed, however has a disadvantage linked to the disadvantageous transmission of the force of tightening of the screw to the blocked element, i.e. to the bar (or bone screw or wire). This transmission takes place in fact by means of an third-class lever made up of the external arm resting on a fulcrum at the elastic joint defined previously. This results in a demultiplication of the force transferred to the element and, in a final analysis, in a need for the surgeon to apply a high torque to the tightening screw in order to ensure satisfactory tightening of the structure.

On the other hand a clamp has been proposed in patent application WO 03/068082 which is structured in such a way as to avoid the disadvantage described above.

This clamp uses, as retaining elements for two connection bars to be blocked, a nut and a washer, translating inside appropriately shaped grooves of the attachments and directly restrained to a tightening screw of the type described previously. These restraining elements are provided with an end projection intended, following tightening of the appropriate screw, to engage on the bars, restraining them in their housings.

Also US 2006/0177263 discloses a clamp featuring a similar arrangement. The preamble of claim 1 is based on this document.

Advantageously, therefore, in this case the load of tightening of the screw is transmitted directly, without the adoption of a disadvantageous lever.

The clamp described here, however, has other disadvantages in relation to the prior art.

In the first place it is made up of as many as five separate elements: the two attachments, the tightening screw and the restraining elements. This structure causes possible difficulties for the end user, as the nut and washer elements, of limited dimensions, can easily be lost, making the entire device unusable, or be dropped, making their reassembly complicated.

Moreover the end projection with a restraining function causes a localised distribution of the load on the connection bar to be blocked, and such a localised distribution causes deformations on the same bar and poor resistance of the joint in relation to possible fatigue stresses.

Patent publication US 2002/0177754 discloses a clamp featuring hinged jaws which are kept into closed position by a compression spring. However, such a configuration has the drawback that the retaining force on the bar is relatively low and not adjustable.

### Summary of the invention

The technical problem at the basis of the present invention is therefore that of supplying a clamp which overcomes all the disadvantages disclosed above in relation to the prior art described.

The abovementioned technical problem is solved by a clamp of the type previously indicated wherein the first attachment comprises a mobile jaw hinged, along an hinging axis perpendicular to the plane of the C-shaped section of the lateral housing, at the free end of the external arm of the first attachment: said mobile jaw comprising a hole orthogonal both to the longitudinal extension of the jaw and to the hinging axis, traversed by the tightening element; the tightening of said tightening element causing a pressure of the mobile jaw on the first connection bar in the direction of the internal arm, thus performing an action of restraining of the first connection bar in the lateral housing.

In this way the following advantage is obtained: the transmission of the force of tightening to the connection bars is obtained by means of the independent mobile jaw.

Advantageously the mobile jaw has a concave surface with teeth designed to restrain by contact the first connection bar inside the lateral housing, said concave surface being counter-shaped in relation to the portion of the first connection bar with which it comes into contact.

The external arm of the first attachment comprises two lateral wings between which the abovementioned mobile jaw is hinged.

This configuration of the external arm and of the jaw elements allows an excellent and efficient distribution of the loads on the bar.

The features and the advantages of the clamp according to the invention will be made clearer by the description, given herein below, of one of its embodiments, given by way of a non-limiting example with reference to the accompanying drawings.

### Brief description of the drawings

Figure 1 shows a side view, sectioned along a central plane, of the clamp for orthopaedic external fixator according to the present invention in a first configuration;
Figure 2 shows a perspective view of the clamp of Figure 1 in a second configuration;
Figure 2A shows a perspective view of the clamp of the present invention in an alternative embodiment;

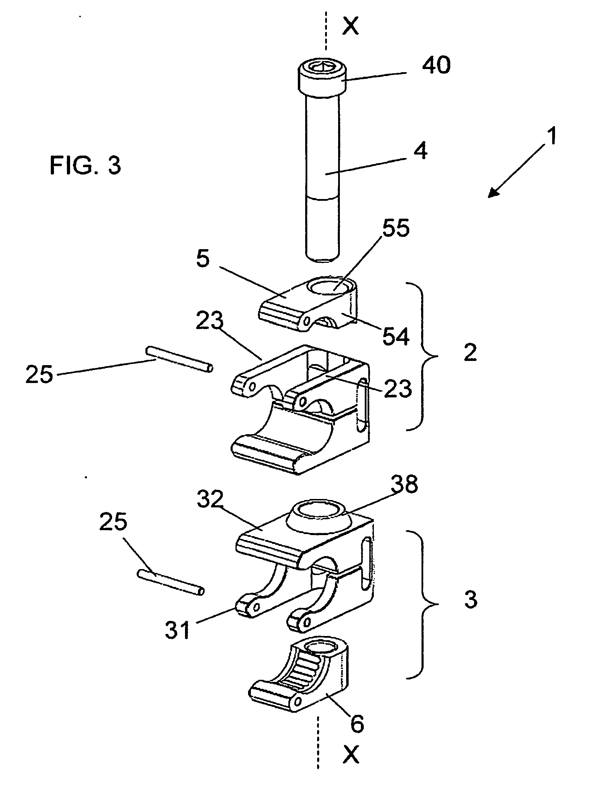

Figure 3 shows a view with detached parts of the clamp shown in Figure 1;
Figure 4 shows a view with detached parts of a clamp for orthopaedic external fixator according to a first embodiment;
Figure 5 shows a view with detached parts of a clamp for orthopaedic external fixator according to a second embodiment;
Figure 6 shows a perspective view of an orthopaedic external fixator incorporating a plurality of clamps according to the embodiments of Figures 1 and 4;
Figures 7 and 8 show respective perspective views of details of the clamp according to the invention in its alternative embodiment of Figure 2A;
Figure 9 shows a perspective view of the clamp of Figure 4 in an alternative embodiment as per Figure 2A.

### Detailed description

Referring to the accompanying drawings, the clamp according to the invention is denoted by reference numerals 1, 1' and 1" according to the reference embodiment.

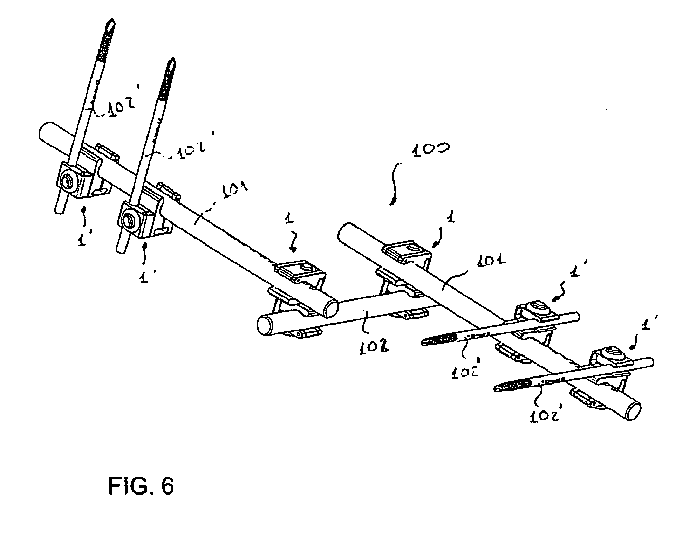

This clamp 1; 1'; 1 ", as explained in the previous discussion on the prior art, is structured to act as a connection element of an orthopaedic external fixator 100, more particularly of a rapid fixator of the type illustrated in Figure 6. This function of the clamp 1, 1', 1" can also be performed temporarily.

The clamp 1; 1'; 1" is meant to create in a rapid and effective manner a temporary rigid connection between a first connection bar 101 and a second component 102; 102' of the fixator 100, for example as shown in Figure 6. In this case three different embodiments of the invention are described in detail, in which the clamp is designed to connect the first connection bar 101 to second components of a different type: in the first embodiment (bar-bar clamp 1) the second component is a second connection bar 102 similar to the first; in the second embodiment (bar-screw clamp 1') it is a bone screw 102'; finally in the third (bar-wire clamp 1") the element connected is a wire (not shown in the drawings).

To allow the performance of their function, the clamps 1; 1'; 1 " comprise advantageously a first attachment 2 and a second attachment 3; 3'; 3", appropriately made integral by a tightening element 4.

As can be seen in the accompanying drawings, the structures of the three clamps are substantially similar and modular, the only differences between them relating to the second attachment 3; 3'; 3": we will therefore continue with a complete description of the bar-bar clamp 1, and then only outline the salient features of the second attachments 3'; 3" which make up the other embodiments. Appropriately, the components of the bar-screw 1' and bar-wire 1" clamps, similar to those of the bar-bar clamp 1, are denoted by the same reference numerals.

The first attachment 2 is provided with two arms, which we will identify respectively as external 21 and internal 22 in relation to the overall structure of the clamp 1 fitted. The arms 21, 22 define one with the other a lateral housing 20 with C-shaped section suitable for accommodating the first connection bar 101.

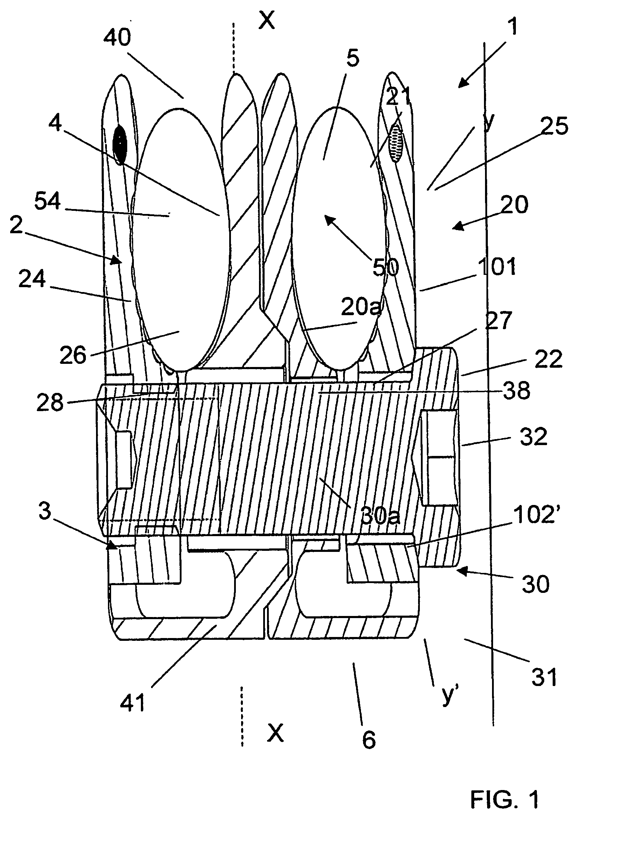

Figure 1 also shows clearly the portion 20a of the housing 20 which is discharged in the centre to ensure proper contact with diameters of bars 101 having different tolerances.

The second attachment 3 of the bar-bar clamp 1, designed to allow connection to a second connection bar 102, has substantially the same structure as the first attachment 2, with the exception of some special features required to obtain the complementarity of the two and which will be described herein below.

The tightening element 4 traverses and connects the first and the second attachment 2, 3 along an axis of rotation x transverse to the extension of the arms 21, 22 mentioned above.

The tightening element 4 is configured like a screw having a head or proximal end 40 with abutment with spherical profile designed to engage by abutting against the surface with bevelled access housing peripheral to a plain hole formed in the first attachment 2, and a tip or distal end 41, threaded and engaged by screwing inside a hole of the second attachment 3.

There is slight play between screw and hole.

The tightening of the tightening element 4 brings the clamp from a loosened configuration, at which the two attachments 2, 3 are reciprocally rotatable around the axis of rotation x, to a tightened configuration, at which the relative orientation of the clamps is blocked.

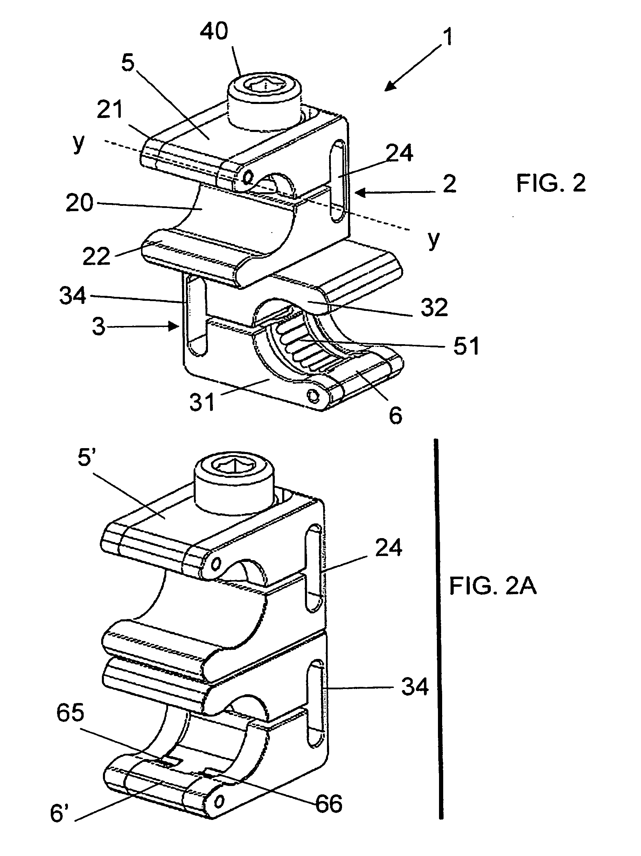

When the clamp 1 is in loosened configuration, it is then possible for the surgeon to orientate the two attachments in the required position. In this respect compare Figure 1, which shows the two attachments in an aligned position, with Figure 2, in which the two attachments are set at a right angle.

As can be seen in the section of Figure 1, the external arm 21 and the internal arm 22 of the first attachment 2 are shaped in such a way as to define a narrowing of the lateral C-shaped housing 20 at their free ends, while they are joined one to the other by an elastic bridge 24 at the opposite ends, so as to allow snap insertion, through said narrowing, of the first connection bar 101 in the lateral housing 20.

The internal arm 22 comprises a central portion 26 traversed by a plain through hole wherein the tightening element 4 is inserted. From this central portion 26 a cantilevered portion 27 leads off in one direction which contributes to defining the lateral housing 20; the elastic bridge 24 mentioned above instead leads off from the opposite direction.

The first attachment 2 comprises also a mobile jaw 5, hinged, along an hinging axis y perpendicular to the plane of the C-shaped section of the lateral housing 20, at the free end of the external arm 21 of the same attachment.

This external arm 21 comprises two lateral wings 23 which extend parallel, forming a fork which constitutes at least the end part but preferably the entire extension of the same arm: hinged between the two lateral wings 23, for example by means of a pin 25 which traverses special holes in the associated elements, is the mobile jaw 5.

The mobile jaw 5 is therefore subjected, according to one of the modes specified herein below in the description, to the tightening element 4 previously introduced, in such a way that the tightening of this element causes a pressure of the mobile jaw 5 on the first connection bar 101 in the direction of the internal arm 21, therefore performing an action of restraining of the first connection bar 101 in the lateral housing 20.

The mobile jaw 5 echoes in part the structure of the internal arm which oppose it. Indeed it comprises, in addition to an end hinging portion 52, a grip portion 53 which is opposite in a substantially symmetrical way to the cantilevered portion 27 of the internal arm 22 and a perforated portion 54 which corresponds to the central portion 26 of said arm. This perforated portion 54 is provided with a hole 55 for the introduction of the tightening element 4, whose axis is orthogonal both to the longitudinal extension of the jaw 5 and to the hinging axis y, and substantially aligned with the axis of the hole of the central portion 26 mentioned.

Special attention should be paid to the shape of the cantilevered portion 27 of the internal arm 22 and that of the opposite grip portion 53 of the mobile jaw 5. The internal surfaces of these parts, in fact, define the C shape of the lateral housing 20 of the first attachment 2. In order to allow an area of extended contact with the first connection bar 101, the portions mentioned thus have concave surfaces, counter-shaped in relation to the portions of connection bar 101 with which they come into contact.

More particularly the concave surface 50 of the mobile jaw 5, designed to restrain by contact the first connection bar 101 inside the lateral housing 20, follows a cylindrical trend, defining a circle arc subtended by an angle preferably slightly greater than 90°, and in any case between 90° and 180°.

Moreover the concave surface 50 of the mobile jaw 5 has indentations 51, preferably transverse and parallel to the hinging axis y, designed to improve the grip of the same jaw 5 on the first connection bar 101.

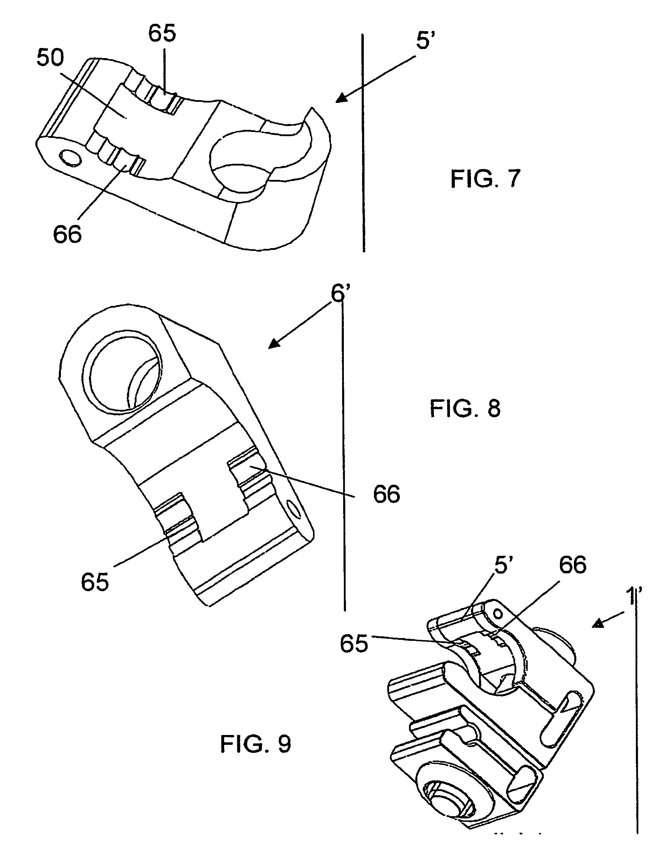

In a preferred embodiment, illustrated for example in Figure 7, it is preferred to adopt a mobile jaw 5' having two shaped profiles with indentations 65, 66 positioned on the lateral edges of the concave surface 50 in a reciprocal distanced relationship. This allows an increase in the friction with the bar 101 and having divided the support in two also confers greater stability.

The hole 55 of the perforated portion of the mobile jaw 54 can be a plain or threaded hole according to the direction of introduction of the tightening element 4.

If the head end 40 (spherical surface) of the tightening element 4 is at the mobile jaw 5, as illustrated in the accompanying drawings, then the bevelled hole 55 will be a plain through hole and the element 4 will be restrained to the jaw by means of the abutment of the head end against an external surface peripheral to this hole. In this case the hole has a diameter such as to grant a slight play between tightening element 4 and jaw, so as to allow easy rotation of the mobile jaw around the hinging axis y.

If instead the tip end 41 of the tightening element 4 has to enter the hole 55, obviously it will be threaded to allow engagement by screwing between the two elements.

The second attachment 3 of the bar-bar clamp 1, as anticipated above, echoes all the features of the first attachment 2, including the housing 30.

Figure 1 also shows very clearly the portion 30a of the housing 30 which is discharged in the centre to ensure proper contact with bar diameters 102' having different tolerances.

This attachment 3 also has a pair of arms 31, 32 at the outermost of which a second mobile jaw 6 is hinged around an hinging axis y'.

The mobile jaw 6 of the second attachment 3, being engaged with the end of the opposite tightening element 4 in relation to the mobile jaw 5 of the first attachment 2, has a threaded hole when the latter has a blind hole, and vice versa.

Otherwise however the mobile jaw 6 corresponds to the structure of the mobile jaw 5.

In a preferred embodiment shown for example in Figure 2 A, the mobile jaws 5 and 6 have been replaced with mobile jaws 5' and 6' with two lateral profiles of indentations 65, 66 rather than a single central profile 51.

One or both the mobile jaws 5' and 6' can also be adopted in any one of the various embodiments which are to be described herein below, for example as illustrated in Figure 9.

The first and the second attachment 2, 3 are associated at their internal arms 22, 32. On their surface of reciprocal contact they have respectively a protuberance 38 and a shaped housing 28, substantially of frustoconical shape, surrounding the passage hole for the tightening element 4 and coaxial thereto. The shaped housing 28 and the protuberance 38 are counter-shaped to ensure a good alignment of the holes of the two parts and to prevent relative sliding during the introduction of the tightening element 4 and increase the tightening torque due to a friction wedge.

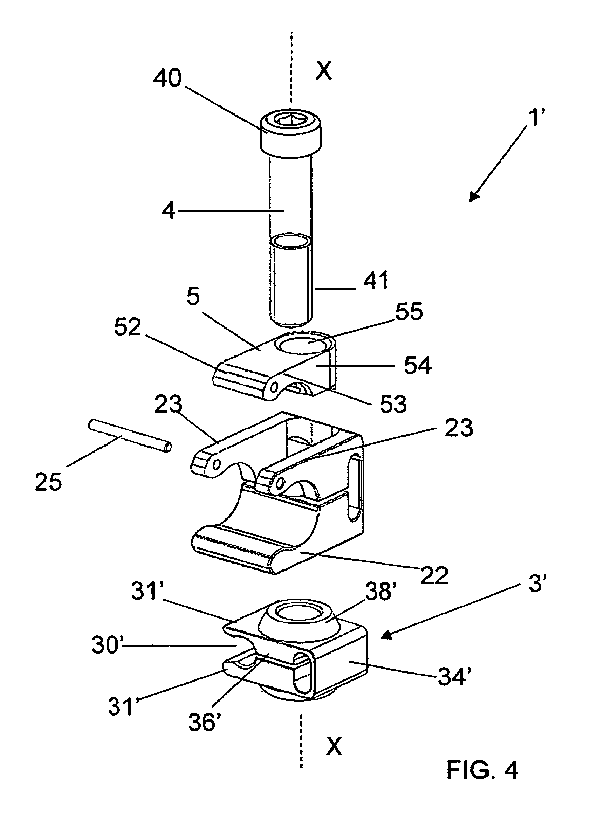

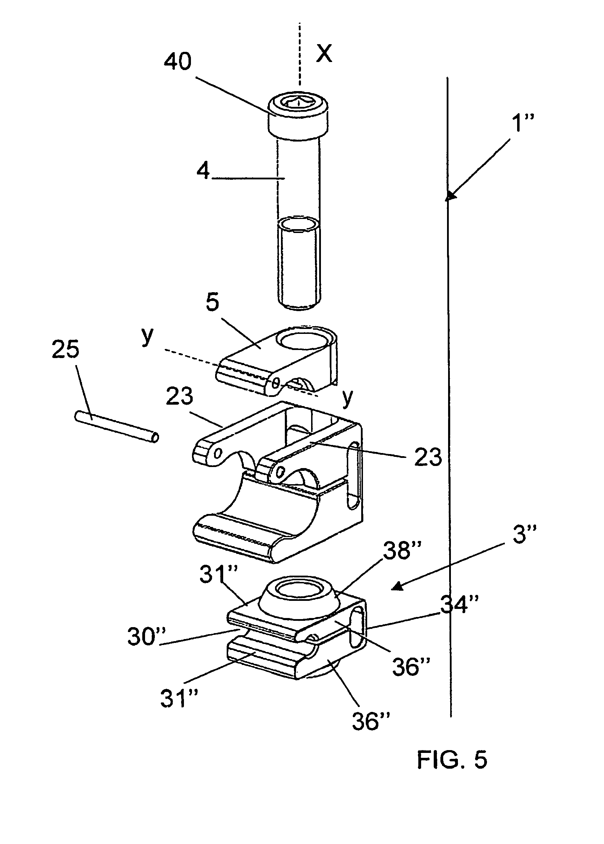

A similar technique can be seen in Figures 4 and 5 also as regards the bar-screw and bar-wire clamps, in which the protuberance 38'; 38" is preferably positioned on the second attachment 3'; 3".

In the embodiments of the bar-screw clamp 1' and of the bar-wire clamp 1 ", illustrated in Figures 4 and 5, the second attachment 3'; 3" takes on a different configuration to restrain respectively a bone screw 102' or wire. The second attachments 3'; 3" relating to said embodiments have moreover the same shape and are only distinguished by the diameter of the lateral housing 30'; 30" intended respectively for housing of the screw 102' or of the wire.

This lateral housing 30'; 30", cylindrical in shape like the element that they are intended to house, are defined by two shaped arms 31'; 31", symmetrically opposite, connected one to the other by an elastic bridge 34'; 34". These arms, like the internal arm 22 of the first attachment 2, comprise a central portion 36'; 36" of increased thickness, traversed by a hole intended for the introduction of the tightening element 4. The element 4 is restrained to the outermost of these arms either by screwing in the hole or by abutment of the head end 40 against an external surface peripheral to it.

The tightening of the tightening element 4 therefore contributes, in addition to binding the two attachments and to blocking the mobile jaw 5 or 5' against the first connection bar 101, to pressing the outermost arm of the second attachment 2'; 2" against the screw 102' or wire in such a way as to block the element into position.

The main advantage of the clamp according to the present invention consists in the advantageous transmission of the force of tightening to the connection bars by means of the independent mobile jaw.

A further advantage arises from the advantageous configuration of the jaw elements, which allows excellent and effective distribution of the loads on the bar.

The indentations provided on the jaw, in the two different embodiments, also contribute to improving the grip of the elements held by the clamp.

Moreover, the insertion of the bars and of the screws in the clamp is perpendicular to the axis of the closure screw.

The closure screw has the support surface with spherical housing. The contact with the surface of the bevel of the hole compensates the deflection of the jaw of the clamp, maintain a correct contact surface.

A further advantage lies in the fact that the clamp is practical and safe to use, being a single piece which can be manipulated without the risk of losing its elements.

Obviously a person skilled in the art, in order to meet contingent and specific needs, may make numerous modifications and changes to the clamp described above within the sphere of protection of the invention as defined by the following claims.

## Claims

1. Clamp (1; 1'; 1") for orthopaedic external fixator (100), comprising:
- a first attachment (2), provided with two arms respectively external (21) and internal (22) in relation to the overall structure of the clamp (1; 1'; 1"), defining between them a lateral housing (20) with C-shaped section suitable for housing a first connection bar (101) of the orthopaedic external fixator (100);
- a second attachment (3; 3'; 3") designed to allow the connection of the clamp (1; 1'; 1") to a second component (102; 102'), other than the first connection bar (101), of the orthopaedic external fixator (100);
- a tightening element (4), at least partially threaded, which traverses and connects the first and the second attachment (2, 3; 3'; 3") along an axis of rotation (x) transverse to the extension of the external and internal arms (21, 22); the tightening of said tightening element (4) bringing the clamp from a loosened configuration, in which the two attachments (2, 3; 3'; 3") are reciprocally rotatable around the axis of rotation (x), to a tightened configuration, in which the relative orientation of the attachments (2, 3; 3'; 3") is blocked;
wherein the first attachment (2; 3; 3'; 3") comprises a mobile jaw (5)
, said mobile jaw (5) comprising a hole (55), orthogonal both to the longitudinal extension of the jaw (5) and to the hinging axis (y), traversed by the tightening element (4); the tightening of said tightening element (4) causing a pressure of the mobile jaw (5) on the first connection bar (101) in the direction of the internal arm (22), performing therefore an action of restraining of the first connection bar (101) in the lateral housing (20); **characterised in that** the mobile jaw is hinged, along an hinging axis (y) perpendicular to the plane of the C-shaped section of the lateral housing (20), at the free end of the external arm (21) of the first attachment (2)

2. Clamp (1; 1'; 1") according to claim 1, wherein the hole (55) is a plain hole and the tightening element (4) has a head end (40) designed to engage by abutting against a surface of the mobile jaw (5) peripheral to said hole (55).

3. Clamp (1; 1'; 1") according to claim 1, wherein the hole (55) is a threaded hole and the tightening element (4) has a threaded tip end (41) which is screwed in said hole (55).

4. Clamp (1; 1'; 1") according to one of the previous claims, wherein the mobile jaw (5) has a concave surface (50) designed to restrain by contact the first connection bar (101) inside the lateral housing (20), said concave surface (50) being counter-shaped in relation to the portion of the first connection bar (101) with which it comes into contact.

5. Clamp (1; 1'; 1") according to claim 4, wherein the concave surface (50) follows a cylindrical trend defining a circle arc subtended by an angle between 90° and 180°.

6. Clamp (1; 1'; 1") according to one of the previous claims, wherein the concave surface (50) has indentations (51) designed to improve the grip of the mobile jaw (5) on the first connection bar (101).

7. Clamp (1; 1'; 1") according to claim 6, wherein the indentations (51) of the concave surface (50) run parallel to the hinging axis (y).

8. Clamp (1; 1'; 1") according to claim 6, wherein the concave surface (50) has indentations (65, 66) positioned at the lateral edges and in a reciprocal distanced relationship.

9. Clamp (1; 1'; 1") according to one of claims 4 to 8, wherein the mobile jaw (5) comprises an end hinging portion (52), a grip portion (53) which has the concave surface (50), and a perforated portion (54) which has the hole (55) for introduction of the tightening element (4), with axis orthogonal both to the longitudinal extension of the jaw (5) and to the hinging axis (y).

10. Clamp (1; 1'; 1") according to claim 9, wherein the external arm (21) comprises two lateral wings (23) between which the mobile jaw (5) is hinged.

11. Clamp (1; 1'; 1") according to one of the previous claims, wherein the external arm (21) and the internal arm (22) of the first attachment (2) are shaped in such a way as to define a narrowing of the lateral C-shaped housing (20) at their free ends, while they are joined one to the other by an elastic bridge (24) at the opposite ends, so as to allow the snap insertion, through said narrowing, of the first connection bar (101) in the lateral housing (20).

12. Clamp (1) according to one of the previous claims, wherein the second attachment (3) is designed to allow the connection of the clamp (1) to a second connection bar (102) of the orthopaedic external fixator (100), and comprises for this purpose two arms respectively external (31) and internal (32), defining between them a lateral housing (30) with C-shaped section suitable for housing the second connection bar (102); and comprises moreover a mobile jaw (6) hinged, along an hinging axis (y') perpendicular to the plane of the C-shaped section of the respective lateral housing (30), at the free end of the external arm (31) and subjected to the tightening element (4) in such a way that a tightening of said tightening element (4) causes a pressure of the mobile jaw (6) on the second connection bar (102) in the direction of the internal arm (31), thus performing an action of restraining of the second connection bar (102) in the lateral housing (30).

13. Clamp (1') according to one of the previous claims, wherein the second attachment (3') is designed to allow the connection of the clamp (1') to a bone screw (102') of the orthopaedic external fixator (100), and comprises for this purpose two shaped arms (31'), symmetrically opposite to define a lateral housing (30') for housing the bone screw (102'), connected one to the other by an elastic bridge (34') and traversed transversely by the tightening element (4) which is restrained to the outermost of these in order to hold it, through tightening of the element (4) against the bone screw (102') so as to block it in the housing (30').

14. Clamp (1") according to one of the previous claims, wherein the second attachment (3") is designed to allow the connection of the clamp (1") to a wire of the orthopaedic external fixator (100) and comprises for this purpose two shaped arms (31"), symmetrically opposite to define a lateral housing (30") for housing the wire, connected one to the other by an elastic bridge (34") and traversed transversely by the tightening element (4) which is restrained to the outermost of these in order to hold it, through tightening of the same element (4) against the wire (102') so as to block it in the housing (30").

15. Clamp (1; 1'; 1") according to one of the previous claims, wherein the reciprocal contact surfaces of the first (2) and of the second attachment (3; 3'; 3") have a counter-shaped protuberance (38, 38', 38") and shaped housing (28) coupled one with the other so as to guarantee correct alignment of the attachments along the axis of rotation (x).

## Patentansprüche

1. Klemme (1; 1'; 1") für eine externe orthopädische Fixiervorrichtung (100), umfassend:
- eine erste Aufhängung (2), die mit zwei Armen, die in Bezug auf die Gesamtstruktur der Klemme (1; 1'; 1") als äußerer Arm (21) und als inneren Arm (22) bezeichnet werden, welche ein laterales Gehäuse (20) mit C-förmigem Querschnitt einschließen, das zur Aufnahme einer ersten Verbindungsstange (101) der externen orthopädischen Fixiervorrichtung (100) ausgebildet ist;
- eine zweite Aufhängung (3; 3', 3"), die zur Verbindung der Klemme (1; 1'; 1") mit einem zweiten Element (102; 102') der externen orthopädischen Fixiervorrichtung (100), welches von der ersten Verbindungsstange (101) unterschiedlich ist, ausgebildet ist;
- ein Spannelement (4), das zumindest teilweise mit einem Gewinde versehen ist und welches die erste und die zweite Aufhängung (2, 3; 3'; 3") entlang einer Rotationsachse (x) durchquert und verbindet, welche quer zu der Erstreckung des äußeren und des inneren Arms (21, 22) angeordnet ist; wobei das Spannen des Spannelementes (4) die Klemme von einer gelösten Stellung, in der die beiden Aufhängungen (2, 3; 3', 3") jeweils um die Rotationsachse (x) drehbar sind, in eine gespannte Stellung bringt, in der die jeweilige Ausrichtung der Aufhängungen (2, 3; 3'; 3") blockiert ist;
- wobei die erste Aufhängung (2, 3; 3', 3") eine bewegliche Backe (5) umfasst, und die mobile Backe (5) ein Loch (55) umfasst, das sowohl zu der Längserstreckung der Backe (5) als auch zu der Scharnierachse (y) senkrecht angeordnet ist und durch das sich das Spannelement (4) erstreckt; wobei das Spannen des Spannelementes (4) einen Druck der beweglichen Backe (5) auf die erste Verbindungsstange (101) in Richtung des inneren Arms (22) hervorruft und dadurch die erste Verbindungsstange (101) in dem lateralen Gehäuse (20) einspannt;
**dadurch gekennzeichnet, dass** die bewegliche Backe entlang einer Scharnierachse (y), die senkrecht zu der Ebene des C-förmigen Querschnitts des lateralen Gehäuses (20) am freien Ende des äußeren Arms (21) der ersten Aufhängung (2) scharnierbar ist

2. Klemme (1; 1'; 1") gemäß Anspruch 1, wobei das Loch (55) ein Durchgangsloch ist und das Spannelement (4) ein Kopfende (40) aufweist, der ausgebildet ist, um an eine Oberfläche der beweglichen Backe (5) im Randbereich des Lochs (55) anstoßend in diese einzugreifen.

3. Klemme (1; 1; 1") gemäß Anspruch 1, wobei das Loch (55) ein mit einem Gewindegang versehenes Loch ist und das Spannelement (4) eine mit einem Gewindegang versehenes Ende (41) aufweist, das in das Loch (55) geschraubt wird.

4. Klemme (1; 1'; 1") gemäß einem der vorhergehenden Ansprüche, wobei die bewegliche Backe (5) eine konkave Oberfläche (50) aufweist, die ausgebildet ist, um durch Berühren die erste Verbindungsstange (101) in dem lateralen Gehäuse (20) zu halten, wobei die konkave Oberfläche (50) eine Gegenform zu dem Teil der ersten Verbindungsstange (101) aufweist, das sie berührt.

5. Klemme (1; 1; 1") gemäß Anspruch 4, wobei die konkave Oberfläche (50) einer zylindrischen Tendenz folgt und einen Kreisbogen beschreibt, der über einen Winkel von 90 bis 180° aufgespannt wird.

6. Klemme (1; 1'; 1") gemäß einem der vorhergehenden Ansprüche, wobei die konkave Oberfläche (50) Eindrücke (51) aufweist, die vorgesehen sind, um den Griff der beweglichen Backe (5) auf die erste Verbindungsstange (101) zu verbessern.

7. Klemme (1; 1; 1") gemäß Anspruch 6, wobei die Eindrücke (51) der konkaven Oberfläche (50) parallel zu der Scharnierachse (y) verlaufen.

8. Klemme (1; 1; 1") gemäß Anspruch 6, wobei die konkave Oberfläche (50) Eindrücke (65, 66) aufweist, die an den Seitenkanten und mit beiderseitigem Abstand angeordnet sind.

9. Klemme (1; 1; 1") gemäß einem der Ansprüche 4 bis 8, wobei die bewegliche Backe (5) ein scharnierbares Ende (52), einen Griffteil (53), das die konkave Oberfläche (50) aufweist und einen perforierten Bereich (54) umfasst, in dem das Loch (55) zum Einführen des Spannelementes (4) umfasst, wobei die Achse sowohl zu der Längserstreckung der Backe (5) als auch zu der Scharnierachse (y) senkrecht ist.

10. Klemme (1; 1; 1") gemäß Anspruch 9, wobei der äußere Arm (21) zwei laterale Flügel (23), zwischen denen die bewegliche Backe (5) scharniert ist, umfasst.

11. Klemme (1; 1'; 1") gemäß einem der vorhergehenden Ansprüche, wobei der äußere Arm (21) und der innere Arm (22) der ersten Aufhängung (2) so ausgebildet sind, dass sie an ihren freien Enden eine Verengung des lateralen C-förmigen Gehäuses (20) definieren, wobei sie an den gegenüberliegenden Enden durch ein elastisches Brückenelement (24) miteinander verbunden sind, um das Einrasten der ersten Verbindungsstange (101) in dem lateralen Gehäuse (20) durch die Verengung zu ermöglichen.

12. Klemme (1) gemäß einem der vorhergehenden Ansprüche, wobei die zweite Aufhängung (3) so ausgebildet ist, dass sie die Verbindung der Klemme (1) zu einer zweiten Verbindungsstange (102) des externen orthopädischen Fixierelementes (100) erlaubt und zu diesem Zweck zwei Arme, einen äußeren Arm (31) und einen inneren Arm (32), umfasst, welche ein laterales Gehäuse (30) mit C-förmigem Querschnitt einschließen, das zur Aufnahme einer zweiten Verbindungsstange (102) geeignet ist; und das darüber hinaus eine bewegliche Backe (6), die entlang einer Scharnierachse (y') , welche senkrecht zu der Ebene des C-förmigen Querschnitts des lateralen Gehäuses 30) angeordnet ist, an dem freien Ende des äußeren Arms (31) scharniert ist und durch das Spannelement (4) derart beaufschlagt wird, dass das Spannen des Spannelementes (4) einen Druck der beweglichen Backe (6) auf die zweite Verbindungsstange (102) in Richtung auf den inneren Arm (31) ausübt, wodurch die zweite Verbindungsstange (102) in dem Gehäuse (30) eingespannt wird.

13. Klemme (1`) gemäß, einem der vorhergehenden Ansprüche, wobei die zweite Aufhängung (3') so ausgebildet ist, dass sie das Verbinden der Klemme (1`) mit einer Knochenschraube (102') der externen orthopädischen Fixiervorrichtung (100) ermöglicht und zu diesem Zweck zwei Arme (31') aufweist, die sich symmetrisch gegenüberliegen und ein laterales Gehäuse (30') zum Aufnehmen der Knochenschraube (102') einzuschließen, und die miteinander durch ein elastisches Brückenelement (34') verbunden sind und von dem Spannelement (4) durchquert werden, das an deren äußeren Ende gespannt ist, um es zu halten, wobei durch Spannen des Elementes (4) gegen die Knochenschraube (102`) es in dem Gehäuse (30') blockiert wird.

14. Klemme (1") gemäß einem der vorhergehenden Ansprüche, wobei die zweite Aufhängung (3") so ausgebildet ist, dass sie die Verbindung der Klemme (1 ") mit einem Kabel der externen orthopädischen Fixiervorrichtung (100) ermöglicht und zu diesem Zweck zwei Arme (31") umfasst, die sich symmetrisch gegenüberliegen, um ein laterales Gehäuse (30") definieren, um das Kabel aufzunehmen, und über ein elastisches Brückenelement (34") miteinander verbunden sind und von dem Spannelement (4) durchquert werden, das an dessen äußeren Ende gespannt ist, um es zu halten, wobei durch Spannen des Elementes (4) gegen das Kabel (102`) es in dem Gehäuse (30") blockiert wird.

15. Klemme (1; 1'; 1") gemäß einem der vorhergehenden Ansprüche, wobei die jeweiligen Kontaktflächen der ersten (2) und der zweiten Aufhängung (3; 3', 3") zueinander passende Vorsprünge (38, 38'; 38") und ein Gehäuse (28) aufweisen, die miteinander gekoppelt sind, um eine korrekte Ausrichtung der Aufhängungen entlang der Rotationsachse (x) zu gewährleisten.

## Revendications

1. Pince (1 ; 1' ; 1") pour fixateur orthopédique externe (100), comprenant :
une première fixation (2) dotée de deux bras respectivement externe (21) et interne (22) par rapport à la structure globale de la pince (1 ; 1' ; 1"), définissant entre eux un logement latéral (20) avec une section en forme de C appropriée pour loger une première barre de raccordement (101) du fixateur orthopédique externe (100) ;
une seconde fixation (3 ; 3' ; 3") conçue pour permettre le raccordement de la pince (1 ; 1', 1") à un second composant (102, 102') différent de la première barre de raccordement (101), du fixateur orthopédique externe (100) ;
un élément de serrage (4), au moins partiellement fileté, qui traverse et raccorde la première et la seconde fixations (2, 3; 3' ; 3") le long d'un axe de rotation (x) transversal par rapport à l'extension des bras externe et interne (21, 22) ; le serrage dudit élément de serrage (4) amenant la pince d'une configuration desserrée dans laquelle les deux fixations (2, 3 ; 3' ; 3") peuvent tourner de manière réciproque autour de l'axe de rotation (x), à une configuration serrée dans laquelle l'orientation relative des fixations (2, 3 ; 3' ; 3") est bloquée ;
dans laquelle la première fixation (2 ; 3 ; 3' ; 3") comprend une mâchoire mobile (5), ladite mâchoire mobile (5) comprenant un trou (55) orthogonal à la fois à l'extension longitudinale de la mâchoire (5) et à l'axe d'articulation (y), traversé par l'élément de serrage (4) ; le serrage dudit élément de serrage (4) provoquant une pression de la mâchoire mobile (5) sur la première barre de raccordement (101) dans la direction du bras interne (22), réalisant par conséquent une action de retenue de la première barre de raccordement (101) dans le logement latéral (20) ;
**caractérisée en ce que** la mâchoire mobile est articulée, le long d'un axe d'articulation (y) perpendiculaire au plan de la section en forme de C du logement latéral (20), au niveau de l'extrémité libre du bras externe (21) de la première fixation (2).

2. Pince (1 ; 1'; 1") selon la revendication 1, dans laquelle le trou (55) est un trou lisse et l'élément de serrage (4) a une extrémité de tête (40) conçue pour se mettre en prise par butée contre une surface de la mâchoire mobile (5) périphérique par rapport audit trou (55).

3. Pince (1 ; 1'; 1") selon la revendication 1, dans laquelle le trou (55) est un trou fileté et l'élément de serrage (4) a une extrémité de pointe filetée (41) qui est vissée dans ledit trou (55).

4. Pince (1; 1' ; 1") selon l'une des revendications précédentes, dans laquelle la mâchoire mobile (5) a une surface concave (50) conçue pour retenir, par contact, la première barre de raccordement (101) à l'intérieur du logement latéral (20), ladite surface concave (50) étant contre-profilée par rapport à la partie de la première barre de raccordement (101) avec laquelle elle vient en contact.

5. Pince (1 ; 1' ; 1") selon la revendication 4, dans laquelle la surface concave (50) suit une tendance cylindrique définissant un arc de cercle sous-tendu selon un angle compris entre 90° et 180°.

6. Pince (1 ; 1'; 1") selon l'une des revendications précédentes, dans laquelle la surface concave (50) a des indentations (51) conçues pour améliorer la préhension de la mâchoire mobile (5) sur la première barre de raccordement (101).

7. Pince (1 ; 1' ; 1") selon la revendication 6, dans laquelle les indentations (51) de la surface concave (50) s'étendent parallèlement à l'axe d'articulation (y).

8. Pince (1 ; 1' ; 1") selon la revendication 6, dans laquelle la surface concave (50) a des indentations (65, 66) positionnées au niveau des bords latéraux et dans une relation de distanciation réciproque.

9. Pince (1 ; 1'; 1") selon l'une quelconque des revendications 4 à 8, dans laquelle la mâchoire mobile (5) comprend une partie d'articulation d'extrémité (52), une partie de préhension (53) qui a la surface concave (50), et une partie perforée (54) qui a le trou (55) pour l'introduction de l'élément de serrage (4), l'axe étant orthogonal à la fois à l'extension longitudinale de la mâchoire (5) et à l'axe d'articulation (y).

10. Pince (1 ; 1'; 1") selon la revendication 9, dans laquelle le bras externe (21) comprend deux ailes latérales (23) entre lesquelles la mâchoire mobile (5) est articulée.

11. Pince (1 ; 1' ; 1") selon l'une des revendications précédentes, dans laquelle le bras externe (21) et le bras interne (22) de la première fixation (2) sont formés de sorte à définir un rétrécissement du logement latéral en forme de C (20) au niveau de leurs extrémités libres, alors qu'ils sont assemblés l'un à l'autre par un pont élastique (24) au niveau des extrémités opposées, afin de permettre l'insertion par encliquetage, à travers ledit rétrécissement, de la première barre de raccordement (101) dans le logement latéral (20).

12. Pince (1) selon l'une des revendications précédentes, dans laquelle la seconde fixation (3) est conçue pour permettre le raccordement de la pince (1) à une seconde barre de raccordement (102) du fixateur orthopédique externe (100) et comprend dans ce but deux bras respectivement externe (31) et interne (32), définissant entre eux un logement latéral (30) avec une section en forme de C appropriée pour loger la seconde barre de raccordement (102) ; et comprend de plus une mâchoire mobile (6) articulée le long d'un axe d'articulation (y') perpendiculaire au plan de la section en forme de C du logement latéral (30) respectif, au niveau de l'extrémité libre du bras externe (31) et soumise à l'élément de serrage (4) de telle sorte qu'un serrage dudit élément de serrage (4) provoque une pression de la mâchoire mobile (6) sur la seconde barre de raccordement (102) dans la direction du bras interne (31), réalisant ainsi une action de retenue de la seconde barre de raccordement (102) dans le logement latéral (30).

13. Pince (1') selon l'une des revendications précédentes, dans laquelle la seconde fixation (3') est conçue pour permettre le raccordement de la pince (1') à une vis à os (102') du fixateur orthopédique externe (100) et comprend dans ce but deux bras profilés (31'), symétriquement opposés afin de définir un logement latéral (30') pour loger la vis à os (102'), raccordés l'un à l'autre par un pont élastique (34') et traversés transversalement par l'élément de serrage (4) qui est retenu le plus à l'extérieur de ces derniers afin de la maintenir, par le biais du serrage de l'élément (4) contre la vis à os (102') de façon à la bloquer dans le logement (30').

14. Pince (1") selon l'une des revendications précédentes, dans laquelle la seconde fixation (3") est conçue pour permettre le raccordement de la pince (1 ") à un fil du fixateur orthopédique externe (100) et comprend dans ce but deux bras profilés(31"), symétriquement opposés afin de définir un logement latéral (30") pour loger le fil, raccordés l'un à l'autre par un pont élastique (34") et traversés transversalement par l'élément de serrage (4) qui est retenu le plus à l'extérieur de ces derniers de façon à la maintenir, par le biais du serrage de ce même élément (4) contre le fil (102') afin de la bloquer dans le logement (30").

15. Pince (1 ; 1'; 1") selon l'une des revendications précédentes, dans laquelle les surfaces de contact réciproques de la première (2) et de la seconde fixations (3 ; 3' ; 3 ") ont une protubérante (38, 38', 38") contre-profilée et le logement profilé (28), couplés l'un à l'autre de façon à garantir le bon alignement des fixations le long de l'axe de rotation (x).
